(19) Europäisches Patentamt
European Patent Office
Office européen des brevets

(11) Publication number: **0 410 393 A2**

# (12) EUROPEAN PATENT APPLICATION

(21) Application number: **90114190.3**

(22) Date of filing: **24.07.90**

(51) Int. Cl.5: **C08L 83/04**, A61K 7/06, D06M 15/643

(30) Priority: **24.07.89 US 383500**
**24.07.89 US 383499**
**24.07.89 US 383509**
**24.07.89 US 383501**

(43) Date of publication of application:
**30.01.91 Bulletin 91/05**

(84) Designated Contracting States:
**AT BE CH DE DK ES FR GB GR IT LI LU NL SE**

(71) Applicant: **AMERICAN CYANAMID COMPANY**
**1937 West Main Street P.O. Box 60**
**Stamford Connecticut 06904-0060(US)**

(72) Inventor: **Villamarin, Arturo A.**
**6430 Brookhills Court S.E. .**
**Grand Rapids Michigan 49546(US)**

(74) Representative: **Wächtershäuser, Günter, Dr.**
**Tal 29**
**D-8000 München 2(DE)**

## (54) Polysiloxane copolymer useful for hair treating.

(57) A novel composition useful to impart semi-permanent properties of conditioning, body and set holding, through repeated hair shampooing; comprising a blend of two reactive silicone polymers as oil-in-water emulsions in acid media, wherein the silicone polymers are a hydroxy terminated dimethyl polysiloxane having the general formula

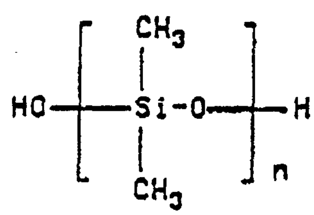

where n is not less than 500,
and

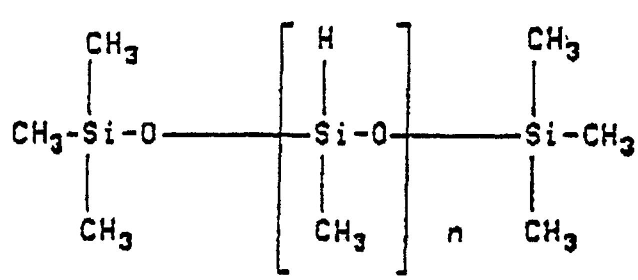

EP 0 410 393 A2

where n is from 15 to 50.

## POLYSILOXANE COPOLYMER USEFUL FOR HAIR TREATING

This invention relates to siloxane copolymer the invention further relates to such a composition useful as a hair treating composition being substantive to the hair after repeated shampooing.

This benefit has been sought in the past and has been achieved to a limited success; for example, by compositions disclosed by Dow Corning, containing amino functional polydimetyl polysiloxanes such as AMODIMETHICONE and TRIMETHYLSILYLAMODIMETHICONE.

A typical high quality hair conditioner such as Revlon's FLEX,, is expected to provide a series of benefits to the hair such as detangling, and low comb drag among others; without imparting an unnatural or greasy feel. The conditioning effect should also not interfere with the setting of the hair such as not allowing the hair to hold the curl or hair style or making the hair stringy or dull a few hours after application by wicking the natural oils from the scalp up the hair shaft.

Hair conditioners formulated with ingredients containing functional groups substantive to the hair such as quaternized proteins, quaternized amines, amine oxides or silicone polymers with amino functional groups to mention a few, tend to be substantive to the hair to varying degrees. In general, the more hydrophobic and the higher the molecular weight of the ingredients; and in the case of molecules carrying a positive charge, the higher their charge densities, the greater their substantivity to the hair.

All the ingredients provide acceptable conditioning benefits to varying degrees, but fail primarily in the area of longer lasting clean feel while providing long lasting conditioning benefits. As a rule of thumb, it can be stated that the greater the substantivity of the conditioning ingredient, the greater the likelihood it will impart a greasy coated feel to the hair or more generally, an unnatural feel and appearance. With some ingredients, over-conditioning and build-up is the result; manifested as the inability of the hair to hold the set and or by having a matted stringy look. The difficulty in balancing residual action with good performance profile without debilitating negative effects, as described, has limited the ability of hair product formulators and marketers to provide a product the consumer does not have to use every time he of she shampoos.

It is the object of this invention to provide long lasting hair conditioning properties without the undesirable effects described before, while giving the user the ability to use it after every shampoo or once a week after four or five shampooings without build-up or over-conditioning while maintaining excellent wet combing and detangling properties. It is also an objective of this invention to provide and conditioner more suitable for use or dry, damaged hair to prevent the incidence of split ends.

According to this invention the objectives are accomplished by treating the hair after shampooing with a composition containing a low viscosity emulsion, less than 200 cps, of a hydroxy terminated dimethyl polysiloxane and a methyl hydrogen polysiloxane, blended at a ratio from about 1:1 to 100:1, being most preferably 10:1. The concentrations of this blend in the formulations being from about 0.5% by weight to active silicone to about 10.0%. The formulated composition has a pH from about two to six ph units, most preferably at about 4.0, which may be adjusted in the usual manner with organic or inorganic acids most preferably with citric acid. This composition may also contain the usual exepients found in most conditioners to provide the desired aesthetic appearance and/or other active conditioning ingredients such as quaternary ammonium salts, amine oxides, fatty alcohol, quaternized or unquaternized proteins, and the like.

Silicones and its derivatives have been used extensively in hair and skin treating preparations, singly, in combination and as copolymers. The composition of this invention consists of a blend of two reactive silicone polymers as oil in water emulsions in acid media.

The hydroxy terminated dimethyl polysiloxane having the general formula:

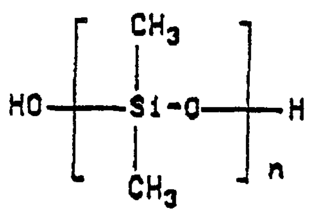

where n is not less than 500,

reacts with a methyl hydrogen polysiloxane having the general formula:

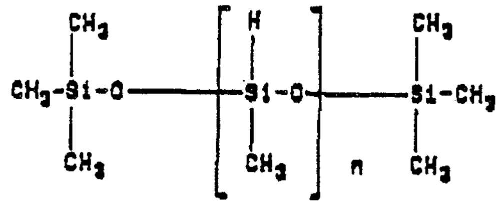

where n is from 15 to 50.

This reaction results in a crosslinked network polymer when an $H_2$ molecule is given off and an oxygen linkage forms between the two molecules. This reaction is well known in the art. Original patent 3,127,636 (wacker-Chemie G.m.b.H) discloses this reaction. Patent 3,637,427 assigned to Nippon Rayon discusses the use of this type composition to treat synthetic fabrics to provide elastic recovery. For this application, the addition of an organometallic catalyst is claimed and a curing temperature between 120°C to 200°C.

It has been found that for the treatment of keratinous substrates (hair and skin) sufficient deposition and adhesion are obtained at temperatures below 100°C, without using a catalyst.

The composition of this invention is useful in reducing the frictional force between the hair and the comb and between hair fibers. This force is generally characterized, in in-vivo testing, as two hair combing parameters; comb drag and detangling. Comb drag - being the frictional force between the comb and the hair; while detangling is associated with the interfiber frictional forces created by the combing action. Untreated tangled hair fibers resist disentangling due to the rough surface of the hair shaft caused by opening and embrittlement of the hair cuticle, as can be appreciated in a micro-photograph of dry or damaged hair.

In order to illustrate the inventions the hair conditioning effects of the composition of Example I, below, were this invention quantified by frictional force measurements on hair tresses, using an Instron instrument. Hair tresses made with natural human hair (30 cm long weighing 2.5 + .2gms) were treated by soaking them for one minute, in the composition of the invention, followed by squeezing out of the excess material prior to drying. The material was not rinsed out, although it may be rinsed out without adversely affecting the conditioning effect or its permanency. A similar set of tresses was treated with a leading commercial conditioner, but the product was rinsed out prior to drying it, as is customary with that type product.

The INSTRON was fitted with a 0.5kg load cell for the test and the test was conducted at a crosshead speed of 20mm/minute, using a comb 15mm wide with average interteeth separations. All measurements were conducted on wet hair.

Referring to the Figures, in Fig. 1 average data is plotted, with the force in grams on the abscissa and the distance travelled by the comb down the hair shaft, on the ordinate; Figure 5 is showns the degreee of damage to hair plotted versus the number of washings. It has been found that combing force measurements made under equal conditions, after the tresses were shampooed once with a commercial shampoo for normal hair, show force differences between the test product and the controls, greater than ten fold in some regions of the hair shaft; particularly at the ends. The single dashed line in the plot shows that for hair treated according to this invention, the force required to comb the tresses barely exceeds 0.01kg for the greater portion of the length of the hair. The noise in the signal or lack thereof, which is associated with the roughness of the hair surface, is also depicted by the flatness of the curve with no significant undulations. In contrast, both plots for the untreated control and the commercial product control are average envelope curves representing extremely jagged curves hard to reproduce in a diagram. These curves show that, after one shampooing, the hair treated with a leading commercial competitive product is just as difficult to comb as combing hair that has never been treated. The treatment is washed out, while the treatment of this invention remains. The conditioning effect of the instant composition was measured up to after six consecutive shampooings. In Fig. 1, only the data for the second and sixth measurements are shown.

In further tests, a statistically designed half head salon test was conducted with 20 subjects, mimicking the protocol used in the force measurement study just described. The produce were applied randomly to the left and right sides of the subject's head and compared for three key wet hair performance attributes and 12 dry hair performance attributes. In addition, seven of these 12 attributes were re-evaluated 24 hours after the treatment. Furthermore, the subjects themselves were given questionnaires focussing on six dry hair evaluations and one overall preference rating.

The results of this study are best understood by following the data shown in Tables 2,3 and 4. The ratings correspond to a three point scale as follows; 0 = no difference, 1 = small difference, 2 = obvious difference and 3 = great difference. Significant differences at the 80%, 90% and 95% confidence level are marked with appropriate superscripts.

The data in Table 2 shows that when the products were first applied the test product was rated significantly (95%) better than the commercial control for three key dry hair conditioning attributes: fewer snarls, less comb drag and smoother feel. All other attributes were basically at parity except for "manageability" for which the commercial control product was found better at the 80% confidence level. Of the wet hair conditioning attributes; less comb drag was superior at the 80% confidence level, while wet detangling was found just directionally better than the leading (FLEX) commercial product. All other attributes were basically at parity except for "manageability" for which the commercial control product was found better at the 80% confidence level.

After the first shampoo when neither one of the products was reapplied, the test product was found superior in several wet and dry conditioning attributes at the 95% confidence level. See column 2 marked Day 2 in Table 2. These data are in complete agreement with the Instron force measurements discussed earlier.

TABLE 1

| OPERATOR'S RATINGS | | | | | |
|---|---|---|---|---|---|
| STANDARD PRE- AND POST-TREATMENT EVALUATION | | | | | |
| | Treatment Differences -Semi-Perm Cond - Flex | | | | |
| | WET EVALUATIONS | | | | |
| Attribute | Initial | Day 2 | Day 3 | Day 4 | Day 5 |
| Detangling (Wet) | .35 | 1.30* | .85 | .85* | .55* |
| Less Comb Drag (Wet) | .55@ | 1.15* | .85* | .70* | .30* |
| Cleaner Feel (After Rinse) | -.10 | -.05 | -.05 | .05 | .05 |
| | DRY EVALUATIONS | | | | |
| Fewer Snarls (Dry) | .85* | 1.35* | .75* | .60* | .25# |
| Less Comb Drag (Dry) | .75* | 1.35* | .55* | .60* | .25# |
| Less Static | -.25 | .30@ | -.05 | .10 | .00 |
| Less Flaking | .00 | .00 | .00 | .00 | .00 |
| More Luster | .20@ | .15 | .05 | .20@ | .05 |
| More Bulk/Fullness | -.05 | .20@ | .10 | -.10 | .00 |
| More Bounce/Spring | -.05 | .50* | .40* | .35* | .05 |
| More Body | .00 | .30* | .25# | .25* | .00 |
| More Manageability | -.25@ | .45* | .05 | .05 | .00 |
| Cleaner Feel (Dry) | -.25 | -.10 | .00 | .05 | .00 |
| Less Tackiness | .00 | .00 | .00 | .00 | .00 |
| Smother Feeling | .70* | .85* | .40* | .25# | .30# |
| @, # and * - difference significant with 80%, 90% or 95% confidence, respectively. | | | | | |

TABLE 2

| OPERATOR'S RATINGS | | | | |
|---|---|---|---|---|
| 24 HOUR EVALUATION | | | | |
| | Treatment Differences -(Semi-Perm Cond -Flex) | | | |
| Attribute | Day 2 | Day 3 | Day 4 | Day 5 |
| Fewer Snarls | .80* | .35# | .50* | .50* |
| Less Comb Drag | .85* | .45* | .30* | .30* |
| More Bulk/Fullness | .20 | .05 | .00 | -.10 |
| More Bounce/Spring | .30# | .20 | .10 | .00 |
| Less Greasy/Cleaner Feel | -.15 | -.10 | .05 | .00 |
| More Curl Retention | .25# | .25@ | .20@ | .00 |
| Better Appearance | .15 | .10 | .20@ | -.05 |
| @. # and * - difference significant with 80%, 90% or 95% *confidence, respectively.* | | | | |

TABLE 3

| SUBJECT'S SELF-EVALUATIONS | | | | | |
|---|---|---|---|---|---|
| AMONG SUBJECTS WITH A PREFERENCE (I.E. IGNORING "NO PREF" RESPONSES), THE TABLE ENTRIES ARE THE PERCENT OF SUBJECTS CHOOSING THE SEMI-PERMANENT HAIR CONDITIONER OVER FLEX HAIR CONDITIONER FOR THE ATTRIBUTE LISTED AT THE LEFT | | | | | |
| % Choosing Semi-Perm Cond over Flex (No Diff = 50%) | | | | | |
| Attribute | Initial | Day 2 | Day 3 | Day 4 | Day 5 |
| After Running Comb/Brush Through Hair: | | | | | |
| Stay in Place Better | 50 | 50 | 63 | 71@ | 56 |
| Looks Fuller/Thicker | 55 | 65@ | 61 | 89* | 56 |
| After Running Fingers Through Hair: | | | | | |
| Stay in Place Better | 50 | 50 | 53 | 71# | 59 |
| Look Fuller/Thicker | 55 | 56 | 40 | 82* | 53 |
| Hair Feels Better | 44 | 35@ | 62 | 62 | 45 |
| Healthier-Looking | 56 | 53 | 64 | 62 | 64 |
| Prefer Overall | 55 | 61 | 55 | 72# | 65@ |
| @, # and * - difference from 50% significant with 80%, 90 or 95% confidence, respectively. The percentage choosing Semi-Permanent Hair Conditioner over FLEX Conditioner is calculated ignoring ties, which number from 0 to 9 of the 20 subjects | | | | | |

For days 3 and 4 these differences persist (after 3 and 4 shampooings) but the significance of the difference wanes by the fifth shampoo, except for wet detangling which is still superior at 95% confidence level and for the dry attributes fewer snarls and less comb drag. Again these data are in strict agreement with the Instron data in Fig. 1.

The evaluation after 24 hours (Table 3) clearly demonstrates an attribute of the present invention and

clearly of support a key claim of this the present invention of hair conditioner product with superior long lasting curl retention properties. This attribute is originally seen, on Table 2, as a composite of two attributes "more bounce/spring" and "more body". The ratings (Table 3) for curl retention correlate with the ratings for fewer snarls (as hair curls fallout while sleeping, the hair gets snarled); particularly taking into consideration that the panelists were not allowed to touch-up their hairdo.

Another attribute of the present invention is the delivery of permanent conditioning benefits without making the hair feel coated, greasy or stiff. In Tables 3, the ratings for cleanliness are consistently at parity with the side which was not treated any further after the first shampoo i.e. the residue on the hair is equal to freshly shampooed hair.

In the third, test the patrons were requested to do a preference evaluation by running the comb or brush through their hair once (without setting it or rearranging the set) for a visual and a tactile evaluation, for five conditioning attributes.

The patron preference results show (Table 4) that their overall preference was for the test product over the five days of the test. It is noteworthy that the number of patrons preferring the test product grew to a maximum at the fourth day. The difference over a split (50/50), was significant at the 90% confidence level, at this point (72/28).

This trend is born out of every single attribute tested, e.g. the preference for "looks fuller/thicker" increased from a 55%/45% split to an 89%/11% at the fourth day. This difference is significant at the 95% confidence level. A similar result was obtained whether the evaluation was done by combing through the hair or by running the fingers through it.

The number of patrons preferring the test product for "(hair) stays in place better, i.e. holding the set" also increased from a 50/50 split to a 71/29; also a significant difference.

The fact that the number of individuals preferring the side of their hair treated with the test product increases with the number of shampooings, could be explained (more testing is required) as a hair protecting mechanism against the harshness of shampooing every day, i.e. as the untreated hair gets stripped further and further by the washing action, the differences between the treated versus the untreated hair increase, until the fourth day. In reality, both sides of the head are getting "damaged" by the shampoo, but at different rates.

This is best understood by examining the diagram of Fig. 2; in it the degree of damage to the hair is plotted versus the number of washings. At day 1 the hair treated with the test product is perceived to be equal in condition or slightly better than the side treated with FLEX, i.e. 8Y is small. After washings two and three the difference between the treatments increases ($8Y_2$ & $8Y_3$) but the differences are not numerically significant. After the fourth washing ($8Y_4$) the difference is maximum and also numerically significant; thereafter the difference begins to narrow ($8Y_5$), ($8Y_6$) etc.

The following specific Examples disclose useful compositions according to the present invention.

| Example I | |
|---|---|
| | % |
| Hydroxy Dimethyl Polysiloxane | 2.000 |
| Methyl Hydrogen Polysiloxane | 0.156 |
| Emulsifying Surfactant | 2.244 |
| Water, DI | 95.600 |
| | 100.000 |

| Example II | |
|---|---|
| Sandoperm, FE | 4.0 |
| Sandoperm, FV | 0.4 |
| Antistatic Agent | 0.5 |
| Fragrance | 0.1 |
| Water, DI | 95.0 |
| | 100.0 |
| ,Sandoz Chemicals Product with improved static control | |

| Example III | | |
|---|---|---|
| | A | B |
| Sandoperm FE | .50 | 1.00 |
| Sandoperm FV | 0.50 | 0.10 |
| Polyurethane QW 4019 | 2.00 | 4.00 |
| SD 40 190 | 77.45 | 64.90 |
| Water, DI | 20.00 | 30.00 |
| | 100.00 | 100.00 |
| Hair Holding Compositions | | |
| The pH of the mixture is adjusted with Citric acid (30%) to pH 4.0 | | |

| EXAMPLE IV | |
|---|---|
| Basic Conditioner | Amount |
| Sandoperm FE Sandoz | 4.000000 |
| Sandoperm FV Sandoz | 0.400000 |
| Water, Deionized | 95.600000 |
| Comments: QS pH to 4.00 with 30% citric acid/cold blend by adding Sandoperms to water. This composition is useful as a conditioner, as such, or in a pump spray package. | |

| EXAMPLE V | |
|---|---|
| Semi-Permanent Conditioner | Amount |
| Sandoperm FE | 4.000000 |
| Sandoperm FV | 0.400000 |
| Fragrance 05-10 | 0.300000 |
| Dye D&C Green # 5 (0.1% in DI. Water | 0.300000 |
| Carbopol 1342 | 0.500000 |
| Water, DI | 94.000000 |
| Comments: Adjust pH to 4.0 with 10% Tea. Viscosity:193.1K 1ps LVT sp.TC,@ 12rpm. This composition is useful as a semi-permanent hair conditioner or creme rinse. | |

| EXAMPLE VI | | |
|---|---|---|
| Product Name: Semi-Permanent Hair Conditioner | | |
| Part No. | Ingredient Name | Amount |
| Sandoperm FE | | 4.000000 |
| Sandoperm FV | | 0.400000 |
| Fragrance 05-10 | | 0.300000 |
| Dye D&C Green #5 (0.1% in DI. Water) | | 0.300000 |
| Xanthan Gum Keltrol Food Grade | | 0.500000 |
| Water, DI | | 94.500000 |
| Comments: Adjust pH 4.0 with 30% Citric Acid. Viscosity 820cps.LVT sp.#2@ 30 rpm. This composition is useful as a semi-permanent hair conditioner or creme rinse. | | |

Further tests were conducted as follows.

Analysis Combined Over Hairtype - (See Tables 4-6)

This study compared the Semi-Permanent Hair Conditioner (SPHC) to Flex Hair Conditioner in a 5-day, "half-head" study in which the SPCH was applied only on the first day while FLEX was applied on each of the 5 days of the study. The purpose was to determine the number of days until the superior conditioning benefits imparted by a single application of SPCH would decline to the level of daily applications of FLEX conditioner.

It should be noted that the salon operators were blinded to the treatments when evaluating but the subjects were not blinded to the treatments on days 2 through 5 since they experienced treatment application on only one side during those days. Thus, a placebo effect may partly account for the fact that the subject's self evaluations tended to favor FLEX on days 2, 3 and (especially) 4. Their evaluations

indicate that "reality" took over by day 5 and the SPHC was again indicated as superior.

Analysis by Hairtype - (See Tables 7-8)

The tables summarize a statistical analysis comparing the 2 hairtypes, "Dry" vs "Normal", in terms of the relative performance of the Semi-Permanent Hair Conditioner (SPHC) and FLEX. Hair Conditioner. Hair classified as Dry is generally colored and/or permed hair and is considered to have some damage. Only the Hair Salon Operator Ratings were analyzed in this way; Self-evaluations were not analyzed by hairtype since to do so would have produced an overly scarce contingency table (see below).

## Statistical Methods

The data included the "Standard Pre- and Post Treatment Evaluations" by the salon operators, "Morning After" Evaluations by the salon operators and "Self-Evaluations" by the subjects. All operators' ratings were analyzed using analysis of variance which accounted for variation due to differences among Hairtypes, differences among subjects within Hairtypes, differences in side of head, differences between the treatments and the interaction of treatments with hairtypes. Subjects' Self-Evaluations were paired choice responses where the subject chose which side had more of each attribute. These data were analyzed using a 2-by-2 table which tabulated subjects by Preferred-Side-of-Head (left vs right) and Treatment/Side-Assignment (A=>Left/B=>Right) vs B=>Left/A=>Right). Subjects' Self-evaluations were not analysed by hairtype since to do so would have produced an overly sparce contingency table: cross classification of 20 subjects by 8 categories (Preferred-Side-of-Head (left vx right), Treatment/Side-Assignment (A=>Left/B=>Right vs B=>Left/A=>Right) and Hairtype (Dry vs Normal)).

Table 4

OPERATOR'S RATINGS

STANDARD PRE- AND POST-TREATMENT EVALUATION

Treatment Differences - (Semi-Perm Cond - Flex)

| Attribute | Initial | Day 2 | Day 3 | Day 4 | Day 5 |
|---|---|---|---|---|---|
| | WET EVALUATIONS | | | | |
| Detangling (Wet) | .62* | .55*. | 1.10* | 1.02* | .76* |
| Less Comb Drag (Wet) | .54* | .78* | 1.10* | 1.19* | .76* |
| Cleaner Feel (After Rinse) | .00 | .12@ | .04 | .00 | .00 |
| | DRY EVALUATIONS | | | | |
| Fewer Snarls (Dry) | .86* | .74* | .83* | .64* | .64* |
| Less Comb Drag (Dry) | .83* | .88* | .83* | .74* | .70* |
| Less Static | .07 | .11 | .05 | .00 | .08 |

| | | | | | |
|---|---|---|---|---|---|
| Better Overall Appearance | .30* | .44* | .43* | .33# | .49* |
| More Luster | .00 | .15 | .20* | .04 | .23* |
| More Bulk/Fullness | .03 | .46* | .11 | .10 | .42* |
| More Bounce/Spring | .15 | .53* | .41* | .14 | .38* |
| More Body | .15 | .59* | .33* | .24@ | .38* |
| More Manageability | .10 | .49* | .23* | .12 | .24@ |
| Cleaner Feel (Dry) | -.01 | .01 | .00 | .00 | .00 |
| More Curl Retention | -.09 | .37* | .12# | -.04 | .16 |
| Smoother Feel | .17# | .25# | .35* | .29* | .21@ |

@, # and * - difference significant with 80%, 90% or 95% confidence, respectively.

Table 5

| OPERATOR'S RATINGS | | | | |
|---|---|---|---|---|
| "MORNING AFTER" EVALUATION | | | | |
| | Treatment Differences - (Semi-Perm Cond - Flex) | | | |
| Fewer Snarls | .53* | .55* | .66* | 1.10* |
| Less Comb Drag | .67* | .55* | .73* | 1.10* |
| Lsss Static | .00 | .00 | -.05 | .00 |
| More Bulk Fullness | -.01 | .22@ | .29* | .19 |
| More Bounce/Springiness | .48* | .40* | .23* | .25@ |
| More Manageability | .37* | .31* | .08@ | .02 |
| Less Greasy (Cleaner Feel) | -.05 | -.13@ | .00 | -.01 |
| More Curl Retention | .35* | .15# | .04 | -.03 |
| Better Appearance .36# | .25 | .56* | .34* | |
| @, # and * - difference significant with 80%, 90% or 95% confidence, respectively. | | | | |

Table 6

| SUBJECT'S SELF-EVALUATIONS | | | | | |
|---|---|---|---|---|---|
| AMONG SUBJECTS WITH A PREFERENCE (L.E. IGNORING "NO PREF" RESPONSES) THE TABLE ENTRIES ARE THE PERCENT OF SUBJECTS CHOOSING THE SEMI-PERMANENT HAIR CONDITIONER OVER FLEX HAIR CONDITIONER FOR THE ATTRIBUTE LISTED AT THE LEFT | | | | | |
| | % Choosing Semi-Perm Cond over Flex (No Diff = 50%) | | | | |
| Attribute | Initial | Day 2 | Day 3 | Day 4 | Day 5 |
| After Running Comb/Brush Through Hair: | | | | | |
| Stays in Place Better | 52 | 56 | 48 | 36@ | 52 |
| Looks Fuller/Thicker | 40 | 48 | 48 | 35@ | 50 |
| After Running Fingers Through Hair: | | | | | |
| Stays in Place Better | 52 | 67@ | 40 | 35@ | 52 |
| Looks Fuller/Thicker | 42 | 54 | 46 | 35@ | 48 |
| Hair Feels Better | 50 | 54 | 46 | 43 | 64@ |
| Healthier-Looking | 36@ | 57 | 32# | 32# | 50 |
| Prefer Overall | 48 | 64@ | 40 | 40 | 54 |
| @, # and * - difference from 50% significant with 80%, 90% or 95% confidence, respectively. The percentage choosing Semi-Permanent Hair Conditioner over Flex Conditioner is calculated ignoring ties, which number from 0 to 9 of the 20 subject . | | | | | |

Table 7

OPERATOR'S RATINGS

STANDARD PRE- AND POST-TREATMENT EVALUATION

BY HAIRTYPE

Treatment Differences - (Semi-Perm Cond. - Flex)

WET EVALUATIONS

| Attribute | Hairtype | Initial | Day 2 | Day 3 | Day 4 | Day 5 |
|---|---|---|---|---|---|---|
| Detangling (Wet) | Dry | .79 | .64 | 1.29 | 1.14 | .71 |
| | Normal | .46 | .45 | .91 | .90 | .80 |
| Less Comb Drag | Dry | .86@ | .94 | 1.29 | 1.07 | .71 |

| | | | | | | |
|---|---|---|---|---|---|---|
| | Normal | .22 | .65 | .91 | 1.30 | .80 |
| Cleaner Feel | Dry | .00 | .14 | .07 | .00 | .00 |
| | Normal | .00 | .09 | .00 | .00 | .00 |

DRY EVALUATIONS

| | | | | | | |
|---|---|---|---|---|---|---|
| Fewer Snarls (Dry) | Dry | 1.21* | .93 | .93 | .57 | .29# |
| | Normal | .50 | .56 | .73 | .70 | .99 |
| Less Comb Drag (Dry) | Dry | 1.14# | .93 | .93 | .79 | .43 |
| | Normal | .52 | .83 | .73 | .70 | .96 |
| Less Static | Dry | .14 | .21 | .00 | .00 | .00 |
| | Normal | .00 | .00 | .09 | .00 | .17 |
| Better Overal Appear. | Dry | .64* | .71# | .57 | .36 | .64 |
| | Normal | -.05 | .18 | .27 | .30 | .34 |
| More Luster | Dry | .07 | .29 | .21 | .07 | .29 |
| | Normal | -.07 | .02 | .18 | .00 | .18 |
| More Bulk/ Fullness | Dry | .36* | .50 | .21 | .00 | .43 |
| | Normal | -.30 | .43 | .01 | .20 | .41 |
| More Bounce/ Spring | Dry | .50* | .79# | .64@ | .07 | .43 |
| | Normal | -.20 | .27 | .18 | .20 | .33 |
| More Body | Dry | .50* | .92* | .57# | .29 | .43 |

| | | | | | | |
|---|---|---|---|---|---|---|
| | Normal | -.20 | .25 | .09 | .20 | .33 |
| More Manageability | | | | | | |
| | Dry | .42* | .71@ | .28 | .14 | .14 |
| | Normal | -.22 | .26 | .18 | .10 | .33 |

Table 4 (continued)

| | | | | | | |
|---|---|---|---|---|---|---|
| Cleaner Feel | Dry | .14 | -.07 | .00 | .00 | .00 |
| | Normal | -.17 | .08 | .00 | .00 | .00 |
| More Curl Retention | Dry | .14 | .57# | .14 | -.07 | .07 |
| | Normal | -.31 | .16 | .09 | .00 | .26 |
| Smoother Feel | Dry | .13 | .22 | .42 | .28 | .14 |
| | Normal | .19 | .28 | .27 | .30 | .27 |

@, # and * - Dry vs Normal Hairtypes significantly different with 80%, 90% or 95% confidence, respectively.

Table 8

| OPERATOR'S RATINGS | | | | | |
|---|---|---|---|---|---|
| "MORNING AFTER" EVALUATION | | | | | |
| | Treatment Differences - (Semi-Perm Cond - Flex) | | | | |
| Attribute | Hairtype | Day 2 | Day 3 | Day 4 | Day 5 |
| Fewer Snarls | Dry | .50 | .86 | .42# | 1.42* |
| | Normal | .55 | .25 | .88 | .75 |
| Less Comb Drag | Dry | .64 | .86* | .50# | 1.42* |
| | Normal | .70 | .25 | .97 | .75 |
| Less Static | Dry | .00 | .00 | .00 | .00 |
| | Normal | .00 | .00 | -.09 | .00 |
| More Bulk/Fullness | Dry | .30 | .36 | .21 | .36 |
| | Normal | -.31 | .08 | .37 | .02# |
| More Bounce/Spring | Dry | .71@ | .64@ | .28 | .50# |
| | Normal | .25 | .17 | .18 | .01. |
| More Manageability | Dry | .71* | .50@ | .07 | .14 |
| | Normal | .03 | .11 | .09 | -.10 |
| Less Greasy | Dry | -.07 | .07* | .00 | .00 |
| | Normal | -.03 | -.33 | .00 | -.01 |
| More Curl Retention | Dry | .57# | .21 | .07 | .14# |
| | Normal | .13 | .08 | .00 | -.17 |
| Better Appearance | Dry | .50 | 1.00* | .14@ | .64@ |
| | Normal | .01 | .12 | .55 | .08 |
| @, # and * - Dry vs Normal Hairtypes significantly different with 80%, 90% and 95% confidence, respectively. | | | | | |

## Claims

1. A stable, room temperature curable water-in-oil emulsion prepolymer blend of the monopolymers
   (1) hydroxy terminated dimethyl polysiloxane having the general formula:

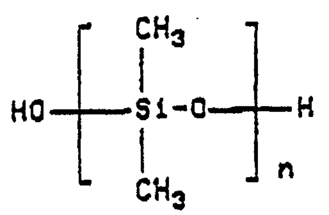

where n is no less than 500,
(2) a methyl hydrogen polysiloxane having the general formula:

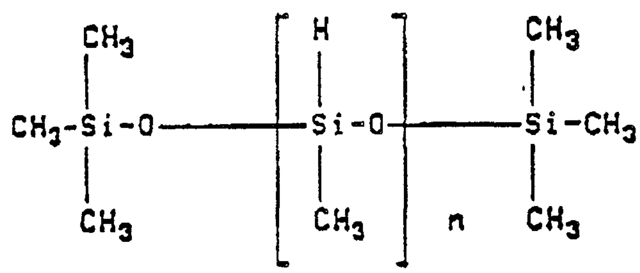

wherein n is from 15 to 50, in an acid medium and water, which form a copolymer in the absence of water.

2. The prepolymer blend of Claim 1 wherein said acid is a low molecular polycarboxylic acid.

3. The prepolymer blend of Claim 2 wherein the amount of acid is sufficient to create a pH of about 2 to 6.

4. The prepolymer blend of Claim 1 wherein the ratio of polysiloxane (1) to (2) is from about 1:1 to 100:1.

5. A composition useful as a coating for a keratinaceous fiber, said composition comprising a stable, room temperature curable water-in-oil emulsion prepolymer blend of the monopolymers

(1) a hydroxy terminated dimethyl polysiloxane having the general formula:

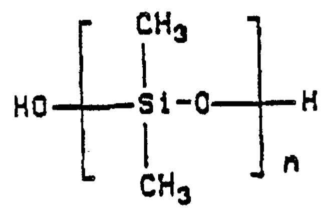

wherein n is no less than 500,

(2) a methyl hydrogen polysiloxane having the general formula:

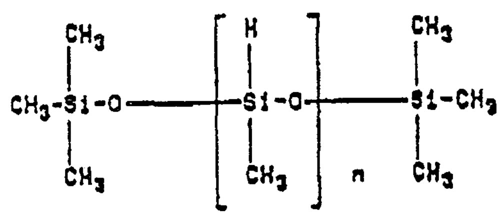

wherein n is from 15 to 50 in an acid medium and water, which form a copolymer in the absence of water, and optionally a detergent, a dye, a thickening agent and a fragrance.

6. The composition of Claim 5 wherein said acid is a low molecular polycarboxylic acid composition.

7. A method for conditioning hair which comprises applying thereto an aqueous water-in-oil emulsion of a room-temperature curable blend of

(1) a hydroxy terminated dimethyl polysiloxane having the general formula:

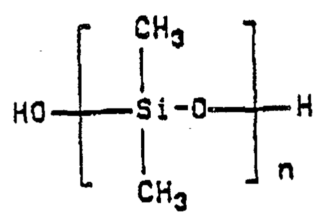

wherein n is no less than 500, and

(2) a methyl hydrogen polysiloxane having the general formula:

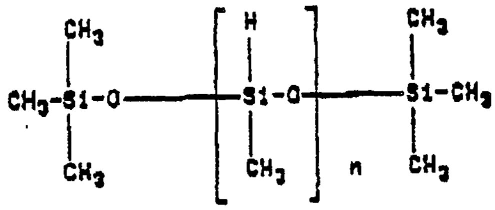

wherein n is from 15 to 50, in an acid medium, and allowing said emulsion to dry on said hair.

8. The method of Claim 7 wherein said evaporation is facilitated by heating at a low temperature.

9. The method of Claim 7 wherein said acid is a low molecular polycarboxylic acid.

10. A method for applying a film of a water insoluble, copolymer formed from two room temperature curable siloxane monopolymers

(1) a hydroxy terminated dimethyl polysiloxane having the general formula:

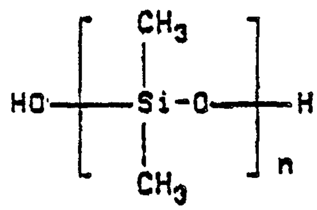

wherein n is no less than 500, and

(2) a methyl hydrogen polysiloxane having the general formula:

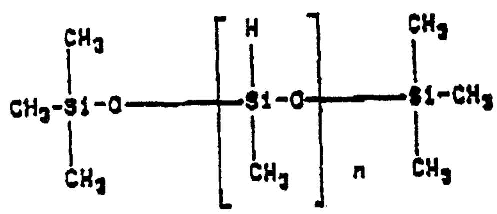

where n is from 15 to 50, wherein said monopolymers are blended with water to form a water-in-oil emulsion, adding a sufficient amount of acid to provide an acid medium, maintaining said emulsion in a non-evaporative state until it is to be applied, applying said emulsion to said fiber, and thereafter allowing said water to evaporate.

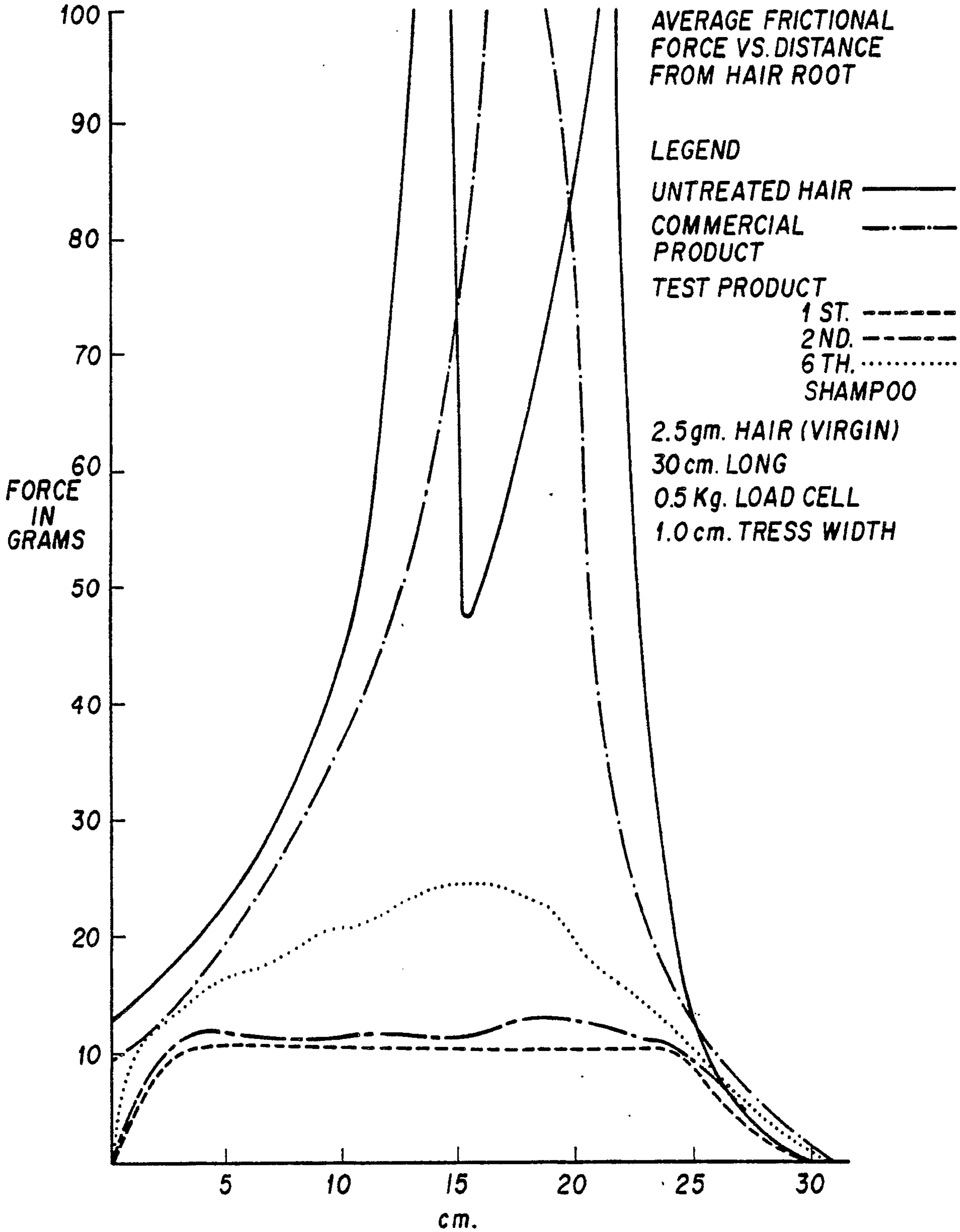
100
90
80
70
60
50
40
30
20
10
FORCE IN GRAMS
5
10
15
20
25
30
cm.
AVERAGE FRICTIONAL FORCE VS. DISTANCE FROM HAIR ROOT
LEGEND
UNTREATED HAIR
COMMERCIAL PRODUCT
TEST PRODUCT
1 ST.
2 ND.
6 TH.
SHAMPOO
2.5 gm. HAIR (VIRGIN)
30 cm. LONG
0.5 Kg. LOAD CELL
1.0 cm. TRESS WIDTH

FIG. 1

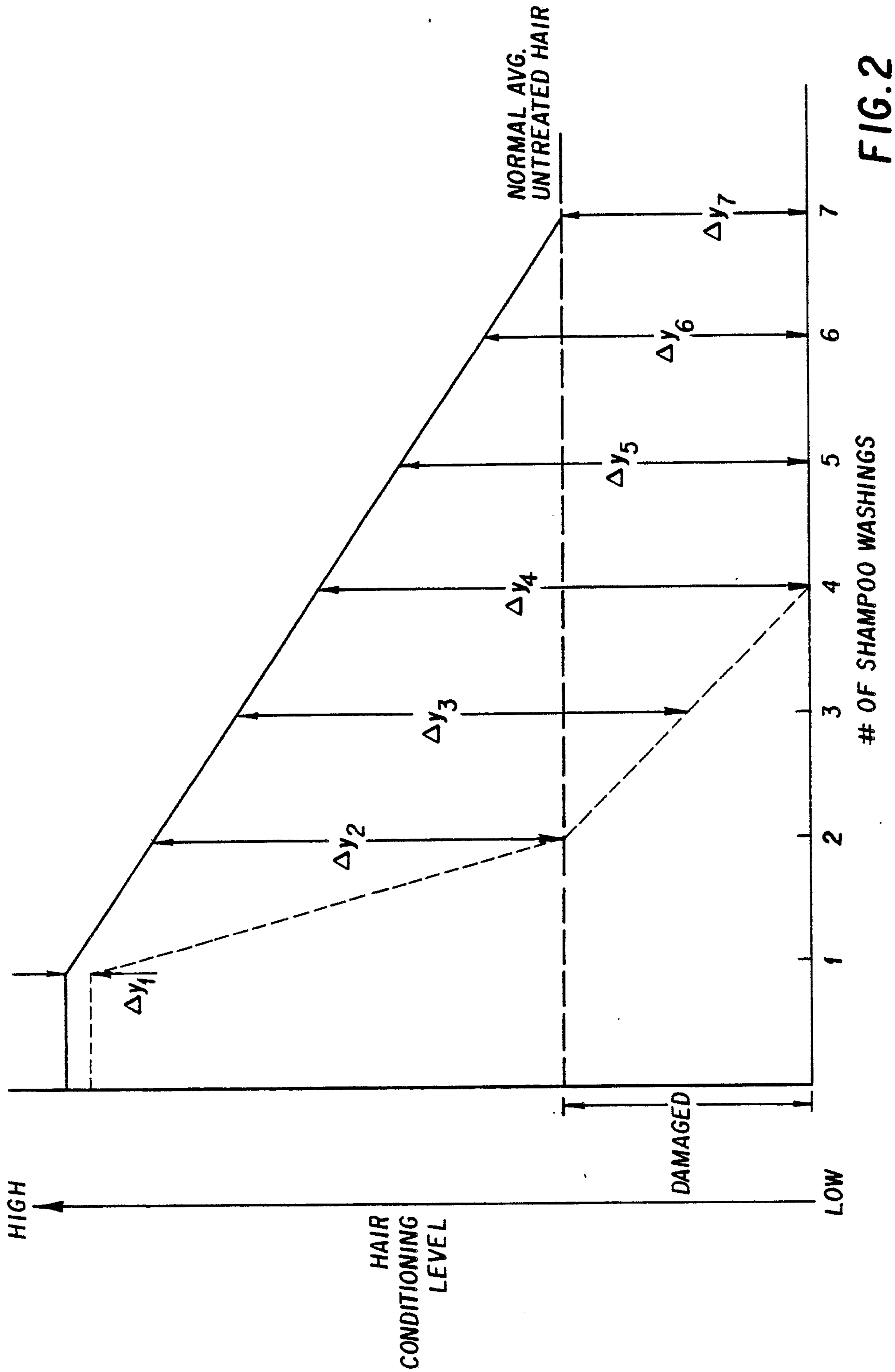
HIGH
HAIR CONDITIONING LEVEL
LOW
DAMAGED
NORMAL AVG. UNTREATED HAIR
Δy1
Δy2
Δy3
Δy4
Δy5
Δy6
Δy7
1
2
3
4
5
6
7
# OF SHAMPOO WASHINGS

FIG.2